# FASCICULE DE BREVET EUROPEEN

(11) **EP 2 953 525 B1**
(45) Date de publication et mention de la délivrance du brevet: **07.09.2022**
(21) Numéro de dépôt: 14705853.1
(22) Date de dépôt: 31.01.2014
(51) Int. Cl.: A61B 5/00

(54) **PROCÉDÉ DE MESURE D'UN PARAMÈTRE PHYSIOLOGIQUE TEL QU'UN RYTHME BIOLOGIQUE À PARTIR D'AU MOINS DEUX CAPTEURS - DISPOSITIF DE MESURE ASSOCIÉ**
VERFAHREN ZUM MESSEN EINES PHYSIOLOGISCHEN PARAMETERS, WIE EINES BIOLOGISCHEN RHYTHMUS, AUF DER BASIS VON MINDESTENS ZWEI SENSOREN UND ZUGEHÖRIGE MESSVORRICHTUNG
METHOD FOR MEASURING A PHYSIOLOGICAL PARAMETER, SUCH AS A BIOLOGICAL RHYTHM, ON THE BASIS OF AT LEAST TWO SENSORS, AND ASSOCIATED MEASUREMENT DEVICE

(30) Priorité: 05.02.2013 FR 1300228
(43) Date de publication de la demande: 16.12.2015
(73) Titulaire: Centre Hospitalier Regional Universitaire de Lille, 59037 Lille Cédex (FR); Société de Ressources et de Développement pour les Entreprises et les Particuliers, 59000 Lille (FR)
(72) Inventeur: LOGIER, Régis, F-59520 Marquette lez Lille (FR); GROSBOIS, Jean-Marie, F-59840 Lompret (FR); DASSONNEVILLE, Alain, F-59116 Houplines (FR); CHAUD, Pascal, F-59211 Santes (FR)
(74) Mandataire: Ipside
(86) Numéro de dépôt international: PCT/FR2014/050173
(87) Numéro de publication internationale: WO 2014/122382

(56) Documents cités:
- US-A1- 2008 167 837
- US-A1- 2010 268 093
- US-A1- 2012 068 848
- R Lukocius ET AL: "The Respiration Rate Estimation Method based on the Signal Maximums and Minimums Detection and the Signal Amplitude Evaluation", Electronics and Electrical Engineering, Kaunaus: Technologija,, 1 janvier 2008 (2008-01-01), pages 51-54, XP055080804, Extrait de l'Internet: URL:http://www.ee.ktu.lt/journal/2008/8/11 _ISSN_1392-1215_The%20Respiration%20Rate%2 0Estimation%20Method%20.pdf [extrait le 2013-09-24]

## Description

La présente invention concerne un procédé de mesure d'un paramètre physiologique tel qu'un rythme biologique ainsi qu'un dispositif de mesure d'un paramètre physiologique pour la mise en œuvre dudit procédé.

La présente invention sera particulièrement destinée à assurer la mesure d'un paramètre physiologique tel que le rythme respiratoire et/ou la fréquence cardiaque d'un individu dans un environnement difficile et par exemple lors de la pratique d'une activité sportive.

Toutefois, bien que particulièrement prévue pour une telle application, la présente invention pourra également être utilisée dans des conditions plus classiques directement par l'individu ou encore par du personnel médical.

On connaît des dispositifs permettant la prise du rythme cardiaque ou encore du rythme respiratoire dans des conditions sportives. Il peut s'agir de dispositifs intégrés directement dans l'équipement sportif comme, par exemple, dans certains vélos d'appartement. Il peut également s'agir de dispositifs indépendants comme les ceintures abdominales qui comportent un capteur de pouls généralement associé à une montre pour afficher en continu le rythme cardiaque, ces ceintures étant classiquement utilisées par les joggeurs et les randonneurs.

En général les dispositifs existants comportent un seul capteur par type de rythme à mesurer. Or la prise de mesure par un seul capteur est très difficile lorsque le dispositif est soumis à des accélérations, à des vibrations ou encore à des chocs dus aux mouvements de l'individu.

Dans la pratique, il est fréquent que, lorsque la perturbation du capteur est trop forte, dans ce cas le dispositif de mesure n'affiche plus de mesure jusqu'à ce que le capteur se stabilise, ou alors il affiche la dernière valeur enregistrée ou enfin il peut afficher des valeurs complètement erronées.

Pour remédier à cela il a été proposé des dispositifs comportant plusieurs capteurs pour mesurer un rythme cardiaque. Ces dispositifs sont plus fiables dans le sens où la probabilité que l'ensemble des capteurs n'assurent pas de mesure est plus faible. Toutefois, le dispositif ne permet pas de discriminer de manière efficace les mesures cohérentes faites par les capteurs des mesures erronées de sorte que la mesure de la fréquence ou du rythme cardiaque est aléatoire ou pour le moins non fiable.

Or dans certains cas la pratique sportive doit être particulièrement surveillée et nécessite des mesures précises. C'est le cas par exemple lorsqu'il s'agit de personnes souffrant de troubles cardiaques ou encore de personnes en rééducation pratiquant des exercices de résistance à l'effort. C'est également le cas lorsque l'individu est un sportif de haut niveau dont les paramètres biologiques doivent être examinés et suivis avec précision.

Des exemples de techniques antérieures sont notamment décrites dans les demandes de brevet américain US2010/268093, US2012/068848 et US2008/167837.

La présente invention constitue un perfectionnement aux dispositifs comportant plusieurs capteurs pour la mesure d'un paramètre physiologique tel que notamment un rythme biologique.

La présente invention a pour but de proposer un procédé de mesure du paramètre physiologique à partir d'au moins deux capteurs dans lequel le paramètre physiologique peut être déterminé de manière fiable et continu même dans un contexte d'acquisition de donnée difficile.

Un autre but de la présente invention est de proposer un procédé de mesure dans lequel les données mesurées sont évaluées en fonction de leur cohérence.

Un autre objet de la présente invention est de proposer un procédé de mesure adapté à la mesure du rythme cardiaque et de la fréquence respiratoire.

Un autre objet de la présente invention est de proposer un dispositif de mesure pour la mise en œuvre du procédé permettant la mesure du rythme cardiaque et de la fréquence respiratoire.

Un autre objet de la présente invention est de proposer un dispositif de mesure intégré à un casque de type casque de vélo.
- A cet effet, l'invention vise un procédé de mesure d'un paramètre physiologique tel qu'un rythme biologique, selon la revendication 1.

L'invention vise également à protéger un dispositif de mesure selon la revendication 5.

La présente invention sera mieux comprise à la lecture ci-après d'un exemple détaillé de réalisation, fourni à titre d'exemple non limitatif et en référence aux figures annexées, parmi lesquelles :
- la figure 1 représente un exemple de réalisation, sous forme schématique, du dispositif de mesure réalisé conformément à la présente invention,
- la figure 2 représente un diagramme représentant différentes étapes du procédé pour la mesure du rythme cardiaque,
- la figure 3 représente un diagramme des différentes étapes d'évaluation de la cohérence d'un signal de fréquence cardiaque,
- la figure 4 représente un diagramme des différentes étapes de sélection de la nouvelle fréquence cardiaque de référence,
- la figure 5 représente un diagramme représentant différentes étapes du procédé pour la mesure de la fréquence respiratoire,
- la figure 6 représente un diagramme des différentes étapes d'évaluation de la cohérence d'un signal de fréquence respiratoire,
- la figure 7 représente un diagramme des différentes étapes de sélection de la nouvelle fréquence respiratoire de référence.

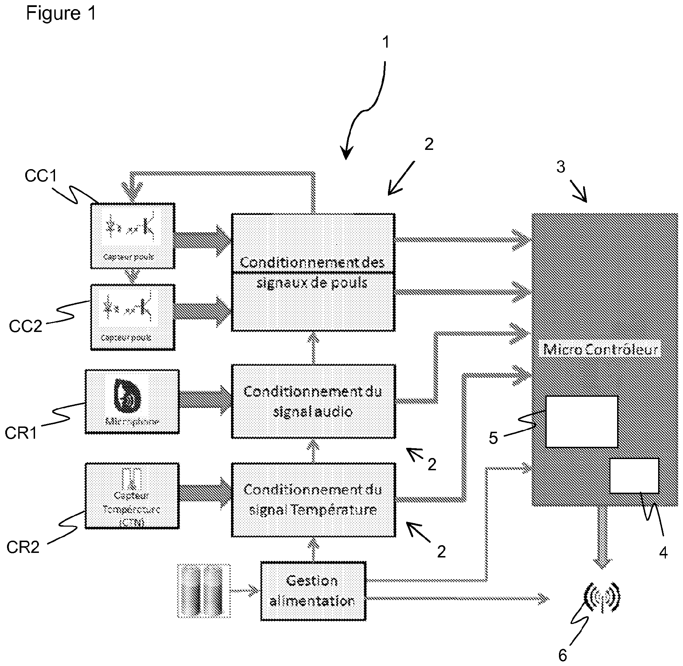

En se reportant principalement à la figure 1, on voit représenté de manière schématique un exemple de réalisation d'un dispositif de mesure 1 pour la mesure d'un rythme biologique. Cet exemple de réalisation permet de bien comprendre l'invention, toutefois il est important de noter que le dispositif ainsi que le procédé ne sont pas limités à la mesure d'un rythme biologique mais s'étendent également à la mesure de tout paramètre physiologique et, par exemple, permettent la mesure de la saturation en oxygène du sang ou encore de la tension artérielle.

Dans l'exemple particulier décrit ci-dessous, s'agissant d'un rythme à mesurer, la mesure faite est celle d'une fréquence, bien évidemment, lorsqu'il s'agira d'autres paramètres physiologiques à mesurer on pourra parler, selon les cas, de taux ou encore de pourcentage ou encore de manière générale de valeur, le principe toutefois de la mesure restant parfaitement identique.

Ce dispositif de mesure 1 permet la mesure de deux rythmes biologique à savoir le rythme cardiaque et la fréquence respiratoire. Dans d'autres modes de réalisation envisageable, le dispositif 1 ne permettra la mesure que d'un seul rythme biologique. Le dispositif de mesure 1 comporte deux capteurs pour la mesure d'un rythme biologique, et par conséquent deux capteurs Cc1 et Cc2 pour la mesure du rythme cardiaque et deux autres Cr1 et Cr2 pour la mesure de la fréquence respiratoire.

Dans l'exemple de réalisation des figures annexées, les deux capteurs Cc1 et Cc2 sont identiques, il s'agit de capteurs de pouls ou oxymétres. Les deux autres capteurs Cr1 et Cr2 sont par contre différents à savoir un capteur Cr1 constitué par un microphone et un capteur Cr2 constitué par un détecteur de température.

L'utilisation de deux capteurs différents pour la mesure d'un rythme biologique présente l'avantage de limiter la probabilité que les deux capteurs ne fonctionnent pas simultanément d'autant que les deux capteurs ne sont pas sensibles aux mêmes types de perturbation tels que les vibrations, les chocs ou encore la température et le taux d'humidité.

Le procédé de mesure associé au dispositif 1 peut toutefois traiter à la fois des capteurs de type différents ou identiques pour l'obtention de mesures fiables. Dans le cas de capteurs identiques on pourra avantageusement prévoir un placement ou positionnement différent des capteurs de sorte que bien qu'identiques, la perturbation d'un des capteurs n'implique pas celle des autres capteurs de même type.

Le dispositif de mesure 1 comporte en outre des moyens de filtrage 2 des signaux émis par les capteurs Cc1, Cc2, Cr1 et Cr2. Ces moyens de filtrage 2 permettent d'éliminer les pics parasites liés aux signaux transmis par les capteurs et de transformer ces signaux de manière à pouvoir les traiter. Le dispositif de mesure 1 comporte également des moyens de traitement 3 des données mesurées. Aux moyens de traitements 3 sont associés des moyens de stockage 4 des données et des moyens d'affichage 5 de la fréquence retenue. Avantageusement les moyens de stockage 3 comportent une mémoire de type mémoire flash. Les moyens d'affichage 5 sont avantageusement constitués par un écran de type LCD.

Il est également important de noter que dans l'exemple de réalisation, le dispositif de mesure 1 comporte des moyens d'émission 6 de données permettant de déporter les moyens d'affichage 5 ou encore de venir afficher les données directement ou de manière supplémentaire sur une unité centrale, non représentée dans les figures annexées.

En outre le dispositif de mesure 1 comprend dans une variante avantageuse des moyens d'alarme permettant une alerte lorsque la fréquence de référence se situe hors d'une plage de valeur.

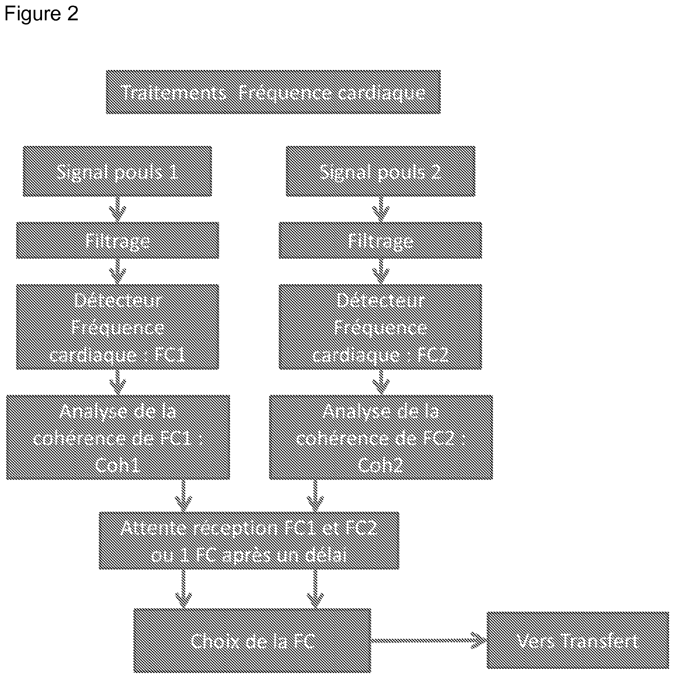

En se reportant cette fois aux figures 2 à 4, on va décrire un premier exemple de fonctionnement du procédé de mesure pour la mesure du rythme cardiaque.

Comme représenté au diagramme de la figure 2 correspondant aux étapes du procédé, la mesure s'effectue à partir des deux capteurs Cc1 et Cc2. Il est important toutefois de noter que le nombre de capteurs peut être plus important et notamment dans le cas où le risque de perte des capteurs est important ou lorsque la fiabilité de ces derniers est limitée.

Le procédé consiste à réaliser une étape de mesure du rythme biologique pour chaque capteur Cc1, Cc2 permettant de générer une série de mesures d'au moins deux fréquences Fc1 et Fc2. Puis le procédé consiste à réaliser une étape d'évaluation du niveau de cohérence de chaque fréquence Fc1, Fc2 de la série de mesures. Cette étape est particulièrement importante puisqu'elle va permettre d'écarter les mesures erronées ou suspectes et de conserver les mesures les plus cohérentes.

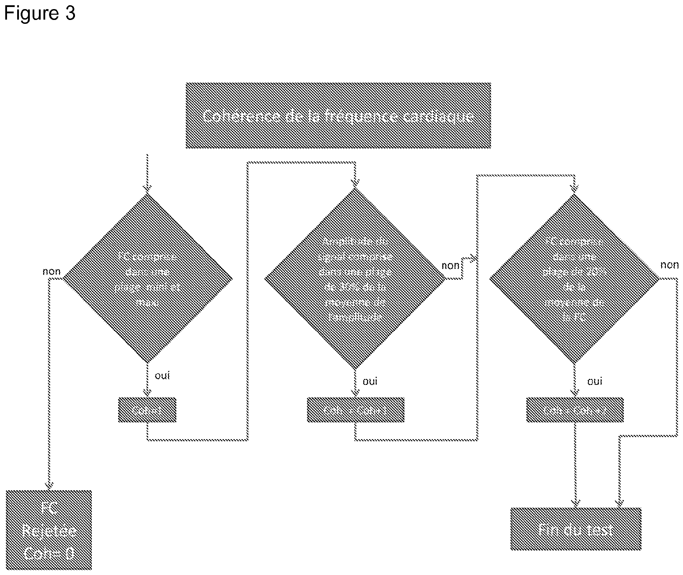

A la figure 3 on voit représenté un diagramme représentant un exemple de réalisation de l'étape d'évaluation. L'étape d'évaluation permet d'attribuer un niveau de cohérence à chaque mesure de la série. A cet effet, l'étape d'évaluation comprend au moins deux examens éliminatoires en série dans lesquels on vérifie si la valeur de chaque fréquence appartient à une plage de valeur. Plus précisément dans cet exemple, l'étape d'évaluation comprend trois examens éliminatoires en série, la réussite à un examen assurant à la fois un point de cohérence à la fréquence mesurée et le passage de l'examen suivant.

Dans ces examens, on vérifie si la fréquence mesurée appartient à une plage de valeur. Dans le premier examen on examine ainsi si la fréquence Fc1mesurée appartient une plage de valeur comprise entre une valeur minimale et maximale, le cas échéant on passe au second examen et on accorde un premier point de cohérence à la fréquence Fc1.

Le second examen consiste à vérifier si le signal possède une amplitude comprise dans une plage correspondant à un pourcentage de la moyenne des amplitudes du signal retenues dites MAR. Le troisième examen consiste à vérifier si la fréquence Fc1 a une valeur comprise dans une plage correspondant à un pourcentage de la moyenne des fréquences retenues dite MFR. Respectivement le pourcentage appliqué est avantageusement de 30% pour le second examen et de 20% pour le troisième examen. La réussite à ces deuxième et troisième examens permet d'accorder un second puis le cas échéant un troisième point de cohérence.

Cette étape d'évaluation est réalisée pour toutes les autres fréquences de la série, c'est à dire les fréquences mesurées par les autres capteurs cardiaques ici Fc2.

Lorsqu'une des fréquences Fc1 ou Fc2 ne reçoit pas de point de cohérence, elle sera écartée lors de l'étape suivante de sélection.

Cette étape de sélection permet de sélectionner une fréquence parmi l'ensemble des fréquences de la série en fonction d'une part de leur niveau de cohérence respectif et d'autre part d'une fréquence dite de référence FR, afin de déterminer une nouvelle fréquence de référence FR.

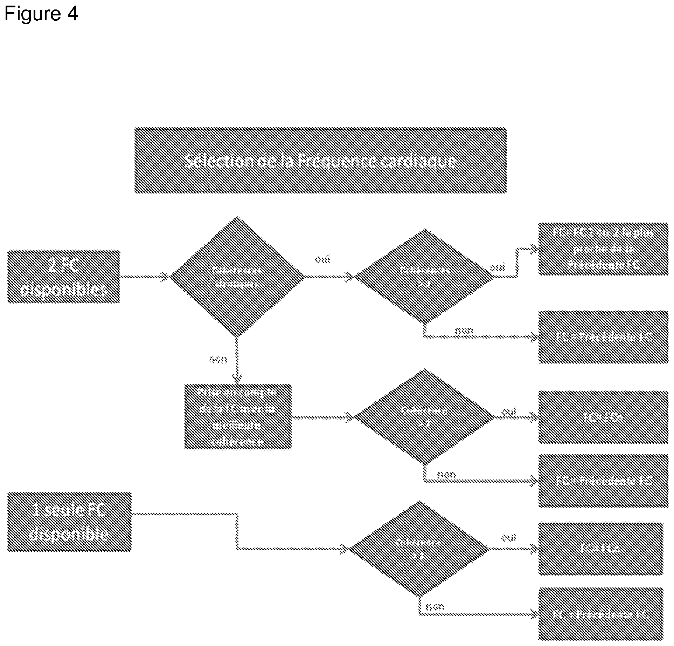

A la figure 4 on voit représenté un diagramme représentant un exemple de réalisation de l'étape de sélection entre les deux fréquences Fc1 et Fc2 d'une série de mesure des deux capteurs Cc1 et Cc2 du rythme cardiaque.

Cette étape comprend une comparaison entre le niveau de cohérence de chaque fréquence de la série de mesures pour conserver uniquement les fréquences présentant le niveau de cohérence le plus élevé de la série. Dans le cas où une fréquence n'est pas disponible on considère que son niveau de cohérence est nul.

Ensuite les moyens de traitement 3 sélectionnent la fréquence la plus proche dite FP de la fréquence de référence dite FR parmi les fréquences présentant le niveau de cohérence le plus élevé.

Une fois la fréquence FP sélectionnée, on réalise le remplacement de la fréquence de référence FR par cette fréquence FP ou le maintien de la fréquence de référence FR si le niveau de cohérence de la fréquence la plus proche FP est inférieur à une valeur seuil.

De manière avantageuse, la fréquence FP sera la nouvelle fréquence de référence FR lorsque le niveau de cohérence atteint sera supérieur ou égal à 2. Bien entendu, cette valeur est modifiable notamment en fonction des points de cohérence accordés à l'issue de chaque examen ou encore en fonction du nombre d'examens pratiqués dans l'étape d'évaluation.

Le procédé comporte également une étape de stockage de la nouvelle fréquence de référence FR dans les moyens de stockage 4.

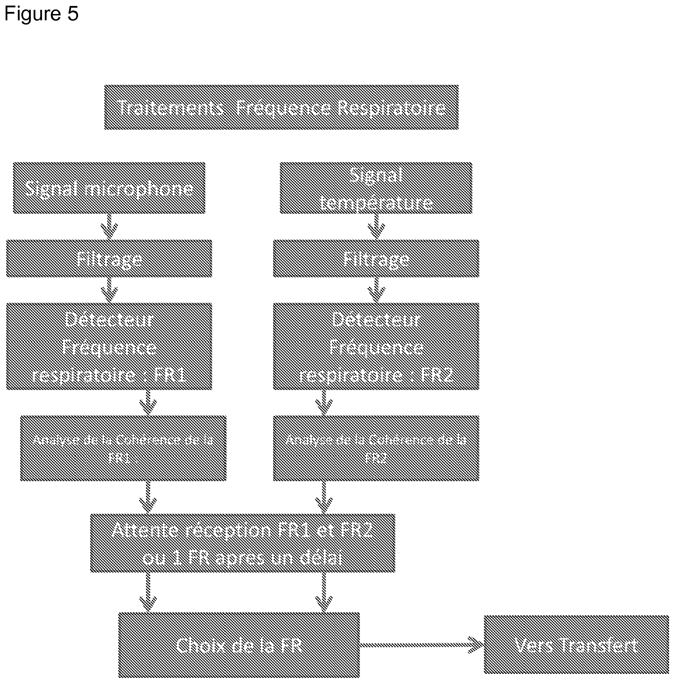

En se reportant cette fois aux figures 5 à 7, on va décrire un premier exemple de fonctionnement du procédé de mesure pour la mesure du rythme respiratoire. Comme représenté au diagramme de la figure 5 correspondant aux étapes du procédé, la mesure s'effectue à partir des deux capteurs Cr1 et Cr2. Là encore, le nombre de capteurs peut être plus important et notamment dans le cas où le risque de perte ou de destruction des capteurs sont élevés.

Dans le cas de la mesure du rythme respiratoire on retrouve les mêmes étapes que pour la mesure du rythme cardiaque avec toutefois des variantes dans l'étape d'évaluation et de sélection.

Il est toutefois important de rappeler que cet exemple est non limitatif et que des variantes de réalisation de ces deux étapes d'évaluation et de sélection sont envisageables.

On retrouve par conséquent dans cette mesure de la fréquence respiratoire une étape de mesure du rythme biologique pour chaque capteur Cr1, Cr2 permettant de générer une série de mesures d'au moins deux fréquences Fr1 et Fr2. Puis on réalise une étape d'évaluation du niveau de cohérence de chaque fréquence Fr1, Fr2 de la série de mesures.

On réalise également l'étape de sélection parmi l'ensemble des fréquences de la série en fonction d'une part de leur niveau de cohérence respectif et d'autre part d'une fréquence dite de référence FR.

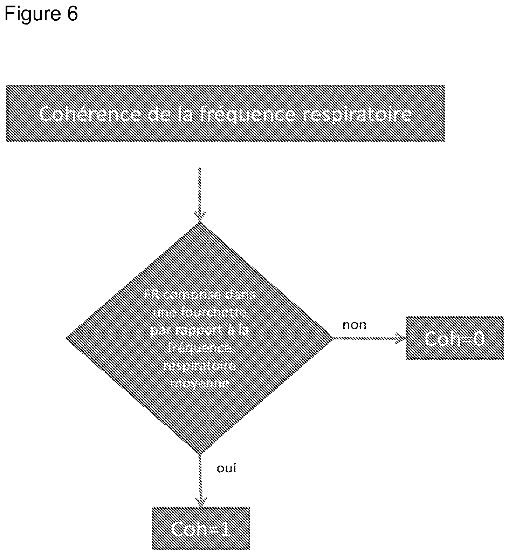

A la figure 6 on voit représenté un diagramme représentant un exemple de réalisation de l'étape d'évaluation. Cette étape est dans l'exemple considérablement simplifiée par rapport à celle prévue dans la mesure du rythme cardiaque. Toutefois, l'objectif de cette étape est identique à savoir permettre l'attribution d'un niveau de cohérence à chaque mesure de la série.

A cet effet, l'étape d'évaluation comprend un examen éliminatoire dans lequel on vérifie si la valeur de chaque fréquence appartient à une plage de valeur. Plus précisément on vérifie si la fréquence Fr1 est comprise dans une plage correspondant à un pourcentage de la moyenne d'amplitude des fréquences respiratoires. Cette étape d'évaluation est réalisée pour toutes les autres fréquences de la série, c'est-à-dire pour les fréquences mesurées par les autres capteurs respiratoires à savoir dans l'exemple Fr2.

Lorsque l'une ou les deux fréquences Fr1 ou Fr2 ne reçoit pas de point de cohérence, elle sera écartée lors de l'étape suivante de sélection.

A l'instar de l'étape de sélection pour la mesure d'un rythme cardiaque, l'étape de sélection permet de sélectionner une fréquence parmi l'ensemble des fréquences de la série en fonction d'une part de leur niveau de cohérence respectif et d'autre part d'une fréquence dite de référence FR, afin de déterminer une nouvelle fréquence de référence FR.

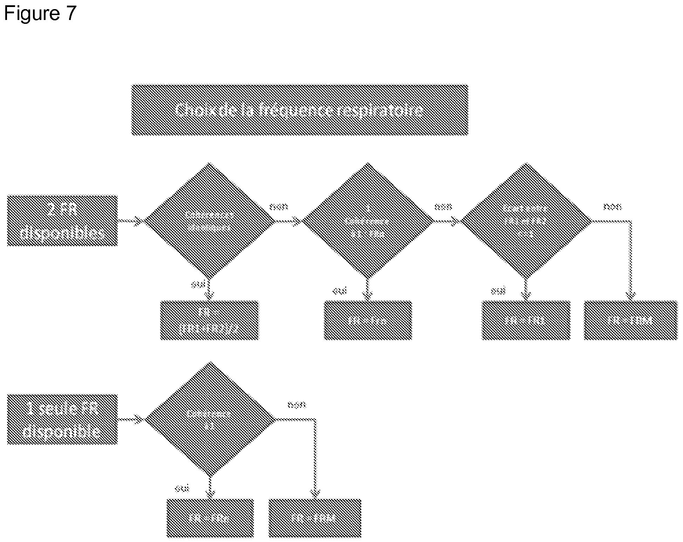

A la figure 7 on voit représenté un diagramme représentant un exemple de réalisation de l'étape de sélection entre les deux fréquences Fr1 et Fr2 d'une série de mesure des deux capteurs Cr1 et Cr2. Cette étape de sélection comprend une comparaison entre le niveau de cohérence de chaque fréquence de la série de mesures pour conserver uniquement les fréquences présentant le niveau de cohérence le plus élevé de la série.

Puis l'étape de sélection comprend un calcul de la moyenne des fréquences parmi les fréquences retenues précédemment. Le procédé consiste ensuite à remplacer la fréquence de référence (FR) par ladite nouvelle moyenne des fréquences ou à maintenir la fréquence de référence (FR) si le niveau de cohérence des fréquences entrant dans le calcul de la moyenne des fréquences est inférieur à une valeur seuil.

Dans le cas où une fréquence Fr1 ou Fr2 ne serait pas disponible on considère que son niveau de cohérence est nul.

Le procédé comporte également une étape de stockage de la nouvelle fréquence de référence FR dans les moyens de stockage 4. Ces derniers stockant à la fois la fréquence de référence FR pour le rythme cardiaque et pour la fréquence respiratoire.

Le dispositif de mesure multi-capteurs et le procédé selon l'invention permettent par conséquent, en associant à une mesure d'un capteur un niveau de cohérence de cette mesure, d'obtenir à l'issue de l'étape de sélection des fréquences un résultat fiable sur la mesure du rythme biologique observé.

Ce dispositif de mesure est par conséquent particulièrement adapté pour la mesure de rythmes biologiques dans des conditions difficiles et plus généralement de tout paramètre physiologique devant être mesuré précisément et ce quelles que soient les conditions de prise des mesures. De manière avantageuse, ce dispositif de mesure sera intégré dans un casque de sorte à être facilement positionné et maintenu sur l'individu.

Bien entendu, d'autres modes de réalisation et variantes à la portée de l'homme de l'Art auraient également pu être envisagés, sans pour autant sortir du cadre de l'invention défini par les revendications ci-après.

## Revendications

1. Procédé de mesure d'un paramètre physiologique tel qu'un rythme biologique à partir d'au moins deux capteurs (Cc1 , Cc2 ; Cr1 , Cr2) dans lequel on réalise la mesure d'un rythme biologique avec :
- une étape de mesure du rythme biologique pour chaque capteur (Cc1, Cc2 ; Cr1, Cr2) permettant de générer une série de mesures d'au moins deux fréquences (Fc1, Fc2 ; Fr1, Fr2),
- une étape d'évaluation du niveau de cohérence de chaque fréquence (Fc1, Fc2 ; Fr1, Fr2) de la série de mesures,
- une étape de sélection d'une fréquence parmi l'ensemble des fréquences de la série en fonction d'une part de leur niveau de cohérence respectif et d'autre part d'une fréquence dite de référence (FR), afin de déterminer une nouvelle fréquence de référence (FR)
- une étape de stockage de la nouvelle fréquence de référence (FR),
et dans lequel on réalise dans l'étape de sélection :
- la comparaison entre le niveau de cohérence de chaque fréquence de la série de mesures pour conserver uniquement les fréquences présentant le niveau de cohérence le plus élevé de la série,
- la sélection de la fréquence la plus proche (FP) de la fréquence de référence (FR) parmi les fréquences présentant le niveau de cohérence le plus élevé,
- le remplacement de la fréquence de référence (FR) par la fréquence de valeur la plus proche (FP) ou le maintien de la fréquence de référence (FR) si le niveau de cohérence de la fréquence la plus proche (FP) est inférieur à une valeur seuil.

2. Procédé de mesure d'un paramètre physiologique selon la revendication 1 dans lequel l'étape d'évaluation comprend au moins deux examens éliminatoires en série dans lesquels on vérifie si la valeur de chaque fréquence appartient à une plage de valeur, l'appartenance à la plage augmentant le niveau de cohérence de la fréquence.

3. Procédé de mesure d'un paramètre physiologique selon la revendication 2 dans laquelle la plage de valeur comprend une valeur basse et une valeur haute correspondant à un pourcentage de la moyenne d'amplitude des fréquences retenues (MFR).

4. Procédé de mesure d'un paramètre physiologique selon l'une quelconque des revendications précédentes, dans lequel on réalise dans l'étape de sélection :
- la comparaison entre le niveau de cohérence de chaque fréquence de la série de mesures pour conserver uniquement les fréquences présentant le niveau de cohérence le plus élevé de la série,
- la moyenne des fréquences parmi les fréquences présentant le niveau de cohérence le plus élevé, - le remplacement de la fréquence de référence (FR) par ladite moyenne des fréquences ou le maintien de la fréquence de référence (FR) si le niveau de cohérence des fréquences entrant dans le calcul de la moyenne des fréquences est inférieur à une valeur seuil.

5. Dispositif de mesure d'un paramètre physiologique tel qu'un rythme biologique pour la mise en œuvre du procédé selon l'une des revendications 1 à 4 précédentes comportant au moins deux capteurs (Cc1, Cc2, Cr1, Cr2) pour la mesure d'un paramètre physiologique, des moyens de filtrage (2) des signaux émis par les capteurs, des moyens de traitement (3) des données mesurées adaptés à la mise en œuvre du procédé selon l'une quelconque des revendications précédentes, des moyens de stockage (4) de données et des moyens d'affichage (5) de la valeur retenue.

6. Dispositif de mesure d'un paramètre physiologique tel qu'un rythme biologique selon la revendication 5 dans lequel au moins deux capteurs de la mesure d'un paramètre physiologique sont de technologies différentes.

7. Dispositif de mesure d'un paramètre physiologique tel qu'un rythme biologique selon l'une ou l'autre des revendication 5 et 6 comportant des moyens d'alarme permettant une alerte lorsque la valeur de référence se situe hors d'une plage de valeur.

8. Dispositif de mesure d'un paramètre physiologique tel qu'un rythme biologique selon l'une ou l'autre des revendications 5 à 7 comportant au moins quatre capteurs et permettant la mesure du rythme cardiaque et de la fréquence respiratoire d'un individu.

## Patentansprüche

1. Verfahren zur Messung eines physiologischen Parameters wie eines biologischen Rhythmus anhand von mindestens zwei Sensoren (Ccl, Cc2; Cr1, Cr2), bei dem die Messung eines biologischen Rhythmus durchgeführt wird mit:
- einem Schritt der Messung des biologischen Rhythmus für jeden Sensor (Ccl, Cc2; Cr1, Cr2), der es ermöglicht, eine Messreihe von mindestens zwei Frequenzen (Fcl, Fc2; Fr1, Fr2) zu erzeugen,
- einem Schritt der Bewertung des Kohärenzniveaus jeder Frequenz (Fcl, Fc2; Fr1, Fr2) der Messreihe,
- einem Schritt der Auswahl einer Frequenz aus allen Frequenzen der Reihe in Abhängigkeit einerseits von ihrem jeweiligen Kohärenzniveau und andererseits von einer Referenzfrequenz (FR), um eine neue Referenzfrequenz (FR) zu bestimmen,
- einem Schritt zur Speicherung der neuen Referenzfrequenz (FR),
und bei dem im Schritt der Auswahl Folgendes durchgeführt wird:
- der Vergleich zwischen dem Kohärenzniveau jeder Frequenz der Messreihe, um nur die Frequenzen zu erhalten, die das höchste Kohärenzniveau der Reihe aufweisen,
- die Auswahl der der Referenzfrequenz (FR) am nächsten liegenden Frequenz (FP) aus den Frequenzen, die das höchste Kohärenzniveau aufweisen,
- das Ersetzen der Referenzfrequenz (FR) durch die Frequenz mit dem am nächsten liegenden Wert (FP) oder die Beibehaltung der Referenzfrequenz (FR), wenn das Kohärenzniveau der am nächsten liegenden Frequenz (FP) unter einem Schwellenwert liegt.

2. Verfahren zur Messung eines physiologischen Parameters nach Anspruch 1, bei dem der Schritt der Bewertung mindestens zwei aufeinanderfolgende Ausscheidungsuntersuchungen umfasst, bei denen überprüft wird, ob der Wert jeder Frequenz zu einem Wertebereich gehört, dessen Zugehörigkeit zu dem Bereich das Kohärenzniveau der Frequenz erhöht.

3. Verfahren zur Messung eines physiologischen Parameters nach Anspruch 2, bei dem der Wertebereich einen niedrigen Wert und einen hohen Wert umfasst, die einem Prozentsatz des Durchschnitts der Amplitude der festgelegten Frequenzen (MFR) entsprechen.

4. Verfahren zur Messung eines physiologischen Parameters nach einem der vorhergehenden Ansprüche, bei dem im Schritt der Auswahl Folgendes durchgeführt wird:
- der Vergleich zwischen dem Kohärenzniveau jeder Frequenz der Messreihe, um nur die Frequenzen zu erhalten, die das höchste Kohärenzniveau der Reihe aufweisen,
- der Durchschnitt der Frequenzen aus den Frequenzen, die das höchste Kohärenzniveau aufweisen,
- das Ersetzen der Referenzfrequenz (FR) durch den Durchschnitt der Frequenzen oder die Beibehaltung der Referenzfrequenz (FR), wenn das Kohärenzniveau der Frequenzen, die in die Berechnung des Durchschnitts der Frequenzen eingehen, unter einem Schwellenwert liegt.

5. Vorrichtung zur Messung eines physiologischen Parameters wie eines biologischen Rhythmus zur Durchführung des Verfahrens nach einem der vorhergehenden Ansprüche 1 bis 4, das mindestens zwei Sensoren (Ccl, Cc2, Cr1, Cr2) zur Messung eines physiologischen Parameters, ein Filtermittel (2) der von den Sensoren emittierten Signale, ein Verarbeitungsmittel (3) der gemessenen Daten, das an die Durchführung des Verfahrens nach einem der vorhergehenden Ansprüche angepasst ist, ein Datenspeichermittel (4) und ein Anzeigemittel (5) des festgelegten Werts umfasst.

6. Vorrichtung zur Messung eines physiologischen Parameters wie eines biologischen Rhythmus nach Anspruch 5, bei der mindestens zwei Sensoren zur Messung eines physiologischen Parameters von unterschiedlicher Technologie sind.

7. Vorrichtung zur Messung eines physiologischen Parameters wie eines biologischen Rhythmus nach einem oder dem anderen der Ansprüche 5 und 6, die ein Alarmmittel umfasst, das einen Alarm ermöglicht, wenn der Referenzwert außerhalb eines Wertebereichs liegt.

8. Vorrichtung zur Messung eines physiologischen Parameters wie eines biologischen Rhythmus nach einem der Ansprüche 5 bis 7, die mindestens vier Sensoren umfasst und die die Messung der Herzfrequenz und Atemfrequenz einer Person ermöglicht.

## Claims

1. A method for measuring a physiological parameter such as a biological rhythm from at least two sensors (Ccl, Cc2; Cr1, Cr2) wherein the measurement of a biological rhythm is performed with:
- a step of measuring the biological rhythm for each sensor (Cc1, Cc2; Cr1, Cr2) for generating a series of measurements of at least two frequencies (Fcl, Fc2; Fr1, Fr2),
- a step of evaluating the level of coherence of each frequency (Fc1, Fc2; Fr1, Fr2) of the series of measurements,
- a step of selecting a frequency from all the frequencies of the series depending on the one hand on their respective level of coherence and on the other hand a so-called reference frequency (FR), in order to determine a new reference frequency (FR),
- a step for storing the new reference frequency (FR), and wherein in the selecting step, there are performed:
- comparing between the level of coherence of each frequency of the series of measurements to keep only the frequencies having the highest level of coherence in the series,
- selecting the nearest frequency (FP) to the reference frequency (FR) from the frequencies having the highest level of coherence,
- replacing the reference frequency (FR) with the nearest value frequency (FP) or maintaining the reference frequency (FR) if the level of coherence of the nearest value frequency (FP) is less than a threshold value.

2. The method for measuring a physiological parameter according to claim 1, wherein the evaluation step comprises at least two eliminatory examinations in series in which it is checked whether the value of each frequency belongs to a range of values, belonging to the range increasing level of coherence of the frequency.

3. The method for measuring a physiological parameter according to claim 2, wherein the range of values comprises a low value and a high value corresponding to a percentage of the amplitude average of retained frequencies (MFR).

4. The method for measuring a physiological parameter according to any of the preceding claims, wherein in the selection step there are performed:
- comparing between the level of coherence of each frequency of the series of measurements to keep only the frequencies having the highest level of coherence in the series,
- averaging the frequencies from the frequencies having the highest level of coherence,
- replacing the reference frequency (FR) with said average of the frequencies or maintaining the reference frequency (FR) if the level of coherence of the frequencies used in averaging the frequencies is less than a threshold value.

5. A device for measuring a physiological parameter such as a biological rhythm for implementing the method according to one of the preceding claims 1 to 4, including at least two sensors (Ccl, Cc2, Cr1, Cr2) for measuring a physiological parameter, means for filtering (2) signals transmitted by the sensors, means for processing (3) measured data adapted to implement the method according to any of the preceding claims, means for storing (4) data and means (5) for displaying the retained value.

6. The device for measuring a physiological parameter such as a biological rhythm according to claim 5, wherein at least two sensors for measuring a physiological parameter are of different technologies.

7. The device for measuring a physiological parameter such as a biological rhythm according to either of claims 5 and 6, including alarm means allowing an alert when the reference value falls outside a range of values.

8. The device for measuring a physiological parameter such as a biological rhythm according to either of claims 5 to 7, including at least four sensors and for measuring the heart rhythm and the respiratory frequency of an individual.
